# EUROPÄISCHE PATENTSCHRIFT

(11) **EP 3 525 687 B1**
(45) Veröffentlichungstag und Bekanntmachung des Hinweises auf die Patenterteilung: **14.05.2025**
(21) Anmeldenummer: 17800363.8
(22) Anmeldetag: 17.10.2017
(51) Int. Cl.: A61B 17/068

(54) **VORRICHTUNG ZUM VERBINDEN VON KÖRPERGEWEBEN**
APPARATUS FOR CONNECTING BODY TISSUES
DISPOSITIF POUR RELIER DES TISSUS CORPORELS

(30) Priorität: 17.10.2016 AT 509362016
(43) Veröffentlichungstag der Anmeldung: 21.08.2019
(73) Patentinhaber: Klaffenböck, Johann, 5350 Strobl (AT); Klaffenböck, Lukas, 5350 Strobl (AT); Mair, Julian, 81925 München (DE)
(72) Erfinder: Klaffenböck, Johann, 5350 Strobl (AT); Klaffenböck, Lukas, 5350 Strobl (AT); Mair, Julian, 81925 München (DE)
(74) Vertreter: Babeluk Patentanwälte GmbH
(86) Internationale Anmeldenummer: PCT/AT2017/060267
(87) Internationale Veröffentlichungsnummer: WO 2018/071935

(56) Entgegenhaltungen:
- EP-A1- 0 040 157
- EP-A1- 0 649 630
- EP-A2- 0 085 931
- WO-A1-2009/135005
- DE-T2- 3 689 806
- US-A- 5 564 615
- US-A1- 2010 276 469

## Beschreibung

Die vorliegende Erfindung betrifft eine Vorrichtung zum Verbinden von Körpergeweben mit einem in eine Körperöffnung einschiebbaren Kopfteil mit einer Längsachse, wobei in dem Kopfteil eine Mehrzahl von Gewebeklammern in einer Vorratsstellung aufgenommen sind, die aus einem linearen Hauptabschnitt und zwei davon senkrecht abstehenden Eingriffsabschnitten bestehen und die jeweils in einer ersten Ebene ausgerichtet sind, die im Wesentlichen senkrecht zur Längsachse ist.

Es ist in der Medizin bekannt, Wunden durch Gewebeklammern zu verschließen, die im Bereich der Wundränder in die Haut eingeführt werden und danach verformt werden, um die Wundränder gegeneinander zu fixieren. Zur Durchführung dieses Vorgangs sind Vorrichtungen entwickelt worden, die im allgemeinen Stapler genannt werden. Solche Vorrichtungen sind beispielsweise in der US 5 170 926 A, der EP 1 695 668 A oder der EP 0 354 724 B beschrieben. Typischerweise werden dabei mehrere Gewebeklammern in einem Magazin gestapelt, ähnlich wie dies bei Büroklammern der Fall ist. Vor der Benutzung wird die jeweils vorderste Gewebeklammern in eine Stellung gedreht, in der sie vorgeschoben und in die Haut eingebracht werden kann. Weitere Vorrichtungen dieser Art sind aus der WO2009/135005 A1, US 5 564 615 A, DE 36 89 806 T2 sowie der EP 0 085 931 A2 bekannt.

Die vorliegende Erfindung betrifft Vorrichtungen, die zur Verbindung von Abschnitten von Körpergeweben in der oben beschriebenen Art geeignet sind, jedoch mit dem Unterschied, dass die Vorrichtung hinreichend klein und kompakt ausgebildet ist, um in künstliche oder natürliche Körperöffnungen eingeführt zu werden, um Körpergewebe im Inneren des Körpers zu verbinden. Es geht also um die Benutzung in endoskopischen oder laparoskopischen Eingriffen. Die besondere Anforderung besteht somit darin, die Vorrichtung mit einem möglichst geringen Querschnitt auszubilden, die erforderlichen Betätigungskräfte sicher einzuleiten und eine hohe Zuverlässigkeit zu gewährleisten, da eine direkte visuelle und taktile Kontrolle bei dieser Form der Anwendung nicht möglich ist.

Es sind verschiedene Vorrichtungen zum Verbinden von Körpergeweben im Inneren des Körpers bekannt geworden. Diese Vorrichtungen sind jedoch aufwändig in der Anwendung und vielfach nur für besondere Anwendungsfälle geeignet.

Aufgabe der vorliegenden Erfindung ist es, eine einfache und problemlos anwendbare Vorrichtung zu schaffen, mit der es möglich ist, Körpergewebe im Inneren des Körpers zu verbinden.

Erfindungsgemäß ist vorgesehen, dass ein Hydraulikzylinder und/oder eine Kolbenstange zumindest teilweise im Inneren des Raums angeordnet sind, der zwischen den Eingriffsabschnitten der Gewebeklammern in der Vorratsstellung liegt, und, dass die Kolbenstange mit dem Schieber fest verbunden ist.

Wesentlich dabei ist zunächst der hydraulische Antrieb über einen Hydraulikzylinder, der unabhängig von der Entfernung zu einem Bedienungselement die Aufbringung der erforderlichen Kräfte ermöglicht. Da die Gewebeklammern in der Vorratsstellung den Hydraulikzylinder bzw. die Kolbenstange teilweise umgeben wird eine besondere Platzersparnis erreicht.

Insbesondere kann die Vorrichtung dadurch besonders kompakt ausgestaltet werden, dass der Hydraulikzylinder doppelwirkend ausgeführt ist.

Erfindungsgemäß sind zwei Rotorelemente vorgesehen, die um eine zu den Hauptabschnitten der Gewebeklammern parallele Achse schwenkbar sind und die jeweils eine Auflagefläche für einen Eingriffsabschnitt einer Gewebeklammer aufweisen, um diese aus der Vorratsstellung in eine Arbeitsstellung zu drehen, in der die Gewebeklammer in einer weiteren Ebene angeordnet ist, die im Wesentlichen senkrecht zur ersten Ebene orientiert ist, und es ist ein Schieber vorgesehen, um die in der Arbeitsstellung vorliegende Gewebeklammer in der weiteren Ebene vorzuschieben und in eine Klammerstellung zu verformen.

Es ist ein wichtiger Aspekt der vorliegenden Erfindung, dass die einzelnen Gewebeklammern aktiv durch Rotorelemente aus der Ebene, in der sie im Magazin gestapelt sind (Vorratsstellung) in die Ebene gedreht werden, in der sie vorgeschoben werden, um in das Körpergewebe einzudringen (Arbeitsstellung).

Die Rotation erfolgt dabei um eine Achse, die senkrecht zur Längsachse der Vorrichtung und zur Vorschubrichtung der Gewebeklammern ist.

Ein weiterer wichtiger Aspekt der vorliegenden Erfindung besteht darin, dass ein Schieber sowohl die Aufgabe übernimmt, eine Gewebeklammer vorzuschieben, als auch die Aufgabe, diese nach dem Eindringen in das Körpergewebe zu verformen, um eine sichere Verbindung herzustellen. Auf diese Weise kann die Anzahl der benötigten Bauteile gering gehalten werden und die Vorrichtung besonders kompakt gestaltet werden.

Es ist günstig, wenn jeweils eine Rotorfeder an einem Rotorelement angreift, um dessen Bewegung in zwei Rotationsstellungen einrastbar auszuführen, und dass vorzugsweise die Rotorelemente mit einer Welle verbunden sind. Auf diese Weise ist es möglich, den Antrieb der Rotorelemente einfach und mit ausreichenden Toleranzen gestalten zu können, da die exakte Winkellage der Rotorelemente in den kritischen Stellungen, nämlich der Aufnahme und der Abgabe der Gewebeklammern durch das Einrasten gewährleistet ist.

Eine besonders elegante Möglichkeit, den Vorschub der Gewebeklammern im Magazin und die notwendige Auflagekraft an den Rotorelementen sicherzustellen besteht darin, dass die Gewebeklammern in Vorratsstellung durch zumindest eine Vorratsfeder über einen Vorratsschlitten gegen das Rotorelement vorgespannt wird, wobei die Vorratsfeder vorzugsweise an der Welle der Rotorelemente befestigt ist. Die Vorratsfeder ist dabei eine Blattfeder, die in die aufgewickelte Stellung vorgespannt ist. Sie hat dabei stets das Bestreben, den linearen Abschnitt parallel zum Magazin zu verkürzen und sich um die Welle aufzuwickeln. Der besondere Vorteil dieser Lösung besteht darin, dass die Kraft, mit der der Vorratsschlitten beaufschlagt wird, unabhängig von der Stellung des Vorratsschlitten ist, also von der ersten bis zur letzten Gewebeklammer im Wesentlichen gleich bleibt.

Ein besonders hoher Grad an Kompaktheit kann dadurch erreicht werden, dass die Rotorelemente durch zumindest ein Pleuel angetrieben werden, das an ihnen angelenkt ist, und welches vorzugsweise über einen Verbindungsschieber mit dem Schieber in Verbindung steht. Auf diese Weise ist es möglich, mit einem einzigen Antriebselement sowohl den Schieber als auch die Rotorelemente anzutreiben, wodurch auch zusätzlicher Aufwand für eine allfällige Synchronisierung unterschiedliche Antriebselemente entfällt.

Insbesondere ist es in diesem Zusammenhang günstig, wenn die Rotorelemente durch je ein Pleuel angetrieben werden, und vorzugsweise je über einen Verbindungsschieber mit dem Schieber in Verbindung stehen. Auf diese Weise wird eine symmetrische Krafteinleitung gewährleistet.

Eine optimale Abfolge der einzelnen Bewegungen wird insbesondere dadurch erreicht, dass der bzw. die Verbindungsschieber fest mit dem Schieber verbunden ist, und dass an jedem Verbindungsschieber ein Langloch angeordnet ist, in das je ein Zapfen des Pleuels eingreift.

Durch das Langloch wird erreicht, dass in einer ersten Phase der Vorwärtsbewegung nur der Schieber vorgeschoben wird, während der Zapfen des Pleuels im Langloch des Verbindungsschiebers nach hinten gleitet, so dass das Pleuel und damit die Rotorelemente nicht bewegt werden. Diese Phase ist dann beendet, wenn der Zapfen am hinteren Ende des Langlochs angekommen ist. In einer zweiten Phase der Vorwärtsbewegung nimmt der Verbindungsschieber nunmehr das Pleuel mit und bewirkt auf diese Weise eine Verdrehung der Rotorelemente.

Bei der Rückwärtsbewegung ist in analoger Weise zunächst nur eine Bewegung des Schiebers zu beobachten, während der Zapfen des Verbindungsschieber im Langloch nach vor gleitet, so dass auch hier keine Bewegung von Pleuel und Rotorelementen stattfindet. Erst dann, wenn der Zapfen am vorderen Ende des Langlochs anliegt, wird das Pleuel mitgenommen und es werden die Rotorelemente zurückgedreht.

Eine besonders bevorzugte Ausführungsvariante der vorliegenden Erfindung sieht vor, dass ein Schieber auf der, den Eingriffsabschnitten abgewandten, Seite der Hauptabschnitte der Gewebeklammern in Richtung der Längsachse beweglich angeordnet ist, der an seiner vorderen Stirnseite zwei seitliche Vorschubbereiche aufweist, zwischen denen eine, im Wesentlichen rechteckige, Ausnehmung vorgesehen ist, und dass ein Rückhalteelement vorgesehen ist, das in die Ausnehmung des Schiebers eingreift, um beim Vorschub des Schiebers den linearen Hauptabschnitt einer dazwischenliegenden Gewebeklammer zu verformen.

Es ist ein wichtiger Aspekt der vorliegenden Erfindung, dass der Schieber sowohl den Vorschub der Gewebeklammern als auch deren Verformung in einer einzigen Bewegung bewerkstelligt. Günstig ist dabei, dass die Kraft auf die jeweilige Gewebeklammer beim Vorschub direkt im Bereich der Eingriffselemente ausgeübt wird, so dass diese gegen den Widerstand des Körpergewebes vorgeschoben werden können, ohne zunächst eine Verformung der Gewebeklammer zu verursachen. Erst dann, wenn die Gewebeklammer ausreichend tief in das Körpergewebe eingedrungen ist, kommt der mittlere Abschnitt des Hauptabschnitts der Gewebeklammer in Anlage an das Rückhalteelement, so dass die Gewebeklammer um die Kanten des Rückhalteelements umgebogen wird.

Nachdem die Gewebeklammer im Körpergewebe ordnungsgemäß gesetzt worden ist, muss eine sichere Ablösung von der erfindungsgemäßen Vorrichtung gewährleistet werden. Gemäß einer bevorzugten Ausführungsvariante der vorliegenden Erfindung erfolgt dies dadurch, dass eine Auswurffeder so angeordnet ist, dass diese eine verformte Gewebeklammer vom Rückhalteelement abstreift. Ein besonderer Vorteil dieser Lösung besteht darin, dass das Abstreifen der Gewebeklammer vollautomatisch geschieht, ohne einen besonderen Steuerungsaufwand zu verursachen.

Das Abstreifen wir den besonders einfache Weise dadurch bewerkstelligt, dass die Auswurffeder im kräftefreiem Zustand in den Bewegungsbereich des Schiebers ragt. Durch das Vorschieben des Schiebers wird die Auswurffeder vorgespannt und durch das Zurückziehen des Schiebers wird die Feder freigegeben, um die Gewebeklammer abzustreifen.

Eine besonders sichere Führung der Gewebeklammer kann dadurch erreicht werden, dass am Schieber eine Nase vorgesehen ist, der die vorzuschiebende Gewebeklammer gegen die Vorspannung der Auswurffeder abstützt.

Eine optimale Konfiguration der Gewebeklammer in appliziertem Zustand wird dadurch erreicht, dass die Ausnehmung im Wesentlichen rechteckig ist.

Eine besonders kompakte und Platz sparende Ausbildung der erfindungsgemäßen Vorrichtung kann dadurch erreicht werden, dass der Schieber plattenförmig ausgebildet ist und der Bewegungsbereich des Schiebers in einer Ebene liegt, die im Bereich der Spitzen der Eingriffsabschnitte der Gewebeklammern in Vorratsstellung liegt, und besonders vorzugsweise im Wesentlichen parallel und mit konstantem Abstand zu den Hauptabschnitten der Gewebeklammern in Vorratsstellung liegt. Auf diese Weise ist es möglich, dass der Schieber und andere Bauteile, die dem Antrieb des Schiebers dienen, den Raum nutzen, der durch die im Magazin im Vorratsstellung vorliegenden Gewebeklammern aufgespannt wird.

Ein besonders effizienter Betrieb der Vorrichtung wird dadurch erreicht, dass der Schieber eine vordere Stellung und eine hintere Stellung besitzt, wobei in der vorderen Stellung zwischen dem Schieber und dem Rückhalteelement eine verformte Gewebeklammer eingeklemmt ist, und in der hinteren Stellung eine aus der Vorratsstellung in eine senkrecht daran liegende Arbeitsstellung gedrehte Gewebeklammer aufnehmbar ist.

Eine besonders begünstigte Ausführungsvariante der Erfindung sieht mindestens einen Kanal zur Aufnahme eines Halteinstruments vor. Solche an sich bekannten Halteinstrumente können vorteilhaft dazu benutzt werden, Gewebe festzuhalten oder in ihrer Lage zu manipulieren, um die Gewebeklammern optimal einsetzen zu können.

Vorzugsweise sind zwei Kanäle vorgesehen, die vorzugsweise nach vorne geringfügig auseinanderlaufen, wobei besonders vorzugsweise der Winkel zwischen den Kanälen steuerbar ist. Dadurch kann entfernteres Gewebe passend herangezogen werden.

Insbesondere kann die Manipulation des Körpergewebes dadurch besonders flexibel gestaltet werden, wenn die Halteinstrumente unabhängig voneinander steuerbar sind.

Eine besonders kompakte Konstruktion der erfindungsgemäßen Vorrichtung kann insbesondere dadurch erreicht werden, dass der Kanal vorzugsweise benachbart zum Hydraulikzylinder angeordnet ist, und/oder dass der Kanal zwischen den Seitenteilen angeordnet ist.

Besonders günstig ist es, wenn der Kanal im proximalen Bereich des Kopfteils angeordnet ist. Dadurch bleibt unterhalb der Gewebeklammern in ihrer Vorratsstellung Platz, so dass sich die Halteinstrumente aus den divergenten Kanälen seitlich auch außerhalb des Querschnitts der Vorrichtung vorgeschoben werden können, um auch laterale Bereiche des Körpergewebes erfassen und in den Bereich der Gewebeklammern ziehen zu können.

In der Folge werden Ausführungsbeispiele der vorliegenden Erfindung anhand der beigefügten Figuren näher erläutert. Es zeigen:
- Fig. 1: eine erste Ausführungsvariante einer erfindungsgemäßen Vorrichtung in einer Ansicht von unten;
- Fig. 2: die Vorrichtung von Fig. 1 in einer Seitenansicht;
- Fig. 3: die Vorrichtung von Fig. 1 und Fig. 2 in einer in einer Ansicht von oben;
- Fig. 4: die Vorrichtung von Fig. 1 bis Fig. 3 in einer in einer Vorderansicht;
- Fig. 5: die Vorrichtung von Fig. 1 bis Fig. 4 in einer Explosionsdarstellung;
- Fig. 6: bis Fig. 9 Längsschnitte in verschiedenen Stadien der Bewegung zur Erklärung der Funktion der Vorrichtung;
- Fig. 10: und Fig. 11 unterschiedliche Schrägansichten einer ersten Ausführungsvariante des Rotorelements;
- Fig. 12: und Fig. 13 unterschiedliche Schrägansichten einer zweiten Ausführungsvariante des Rotorelements;
- Fig. 14: eine weitere Ausführungsvariante einer erfindungsgemäßen Vorrichtung in einem Längsschnitt;
- Fig. 15: und Fig. 16 zwei alternative Einsatzmöglichkeiten der Vorrichtung jeweils in einer Schrägansicht;
- Fig. 17: und Fig. 18 jeweils einen Längsschnitt einer weiteren Ausführungsvariante der Erfindung in zwei unterschiedlichen Stellungen; und

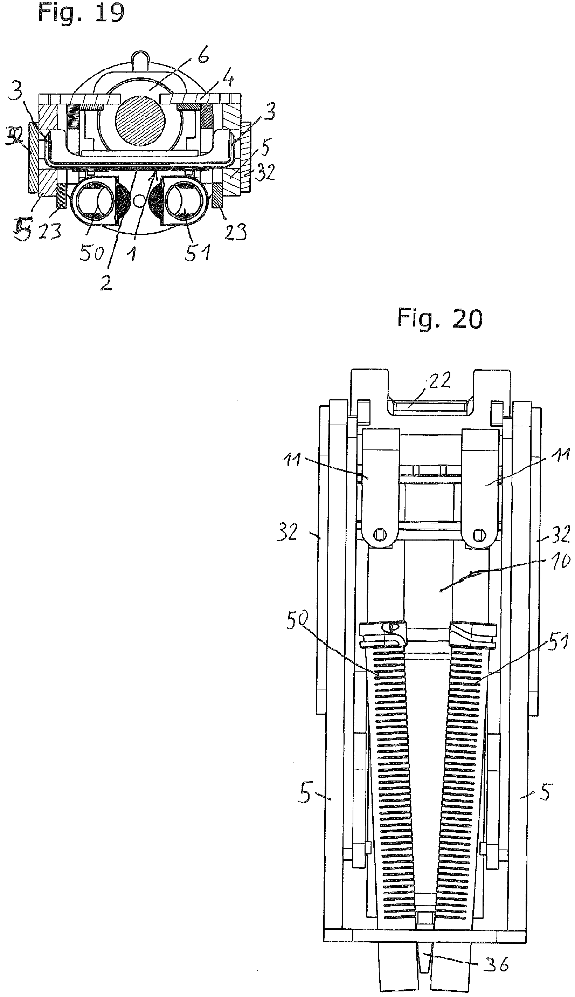

- Fig. 19: einen Querschnitt durch die Ausführungsvariante von Fig. 17 und Fig. 18,
- Fig. 20: eine Ansicht der Ausführungsvariante von Fig. 17 bis Fig. 19 von unten.

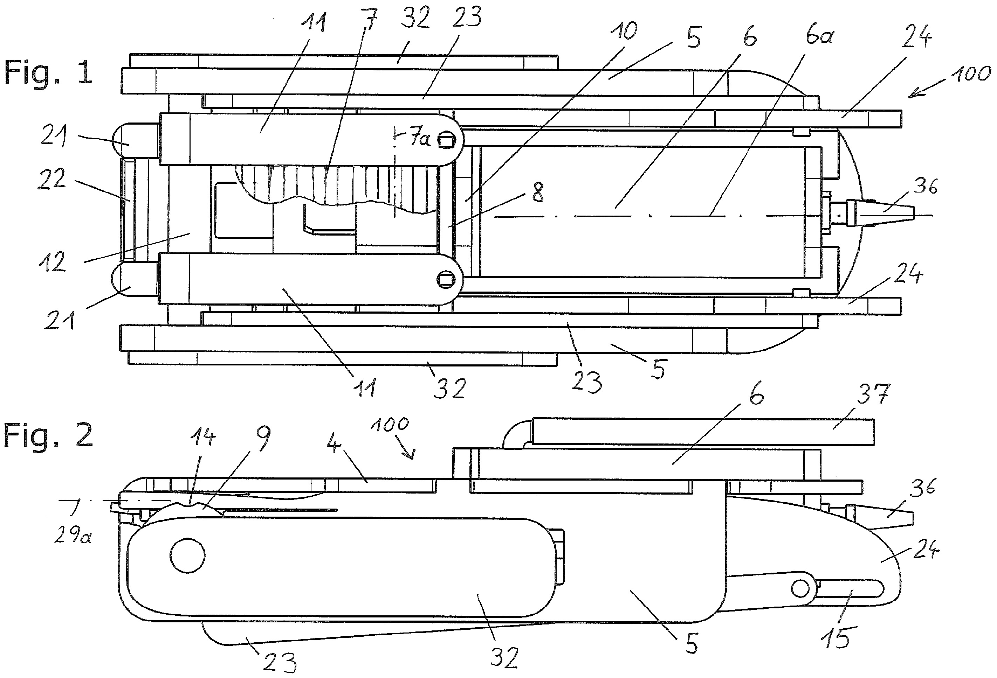

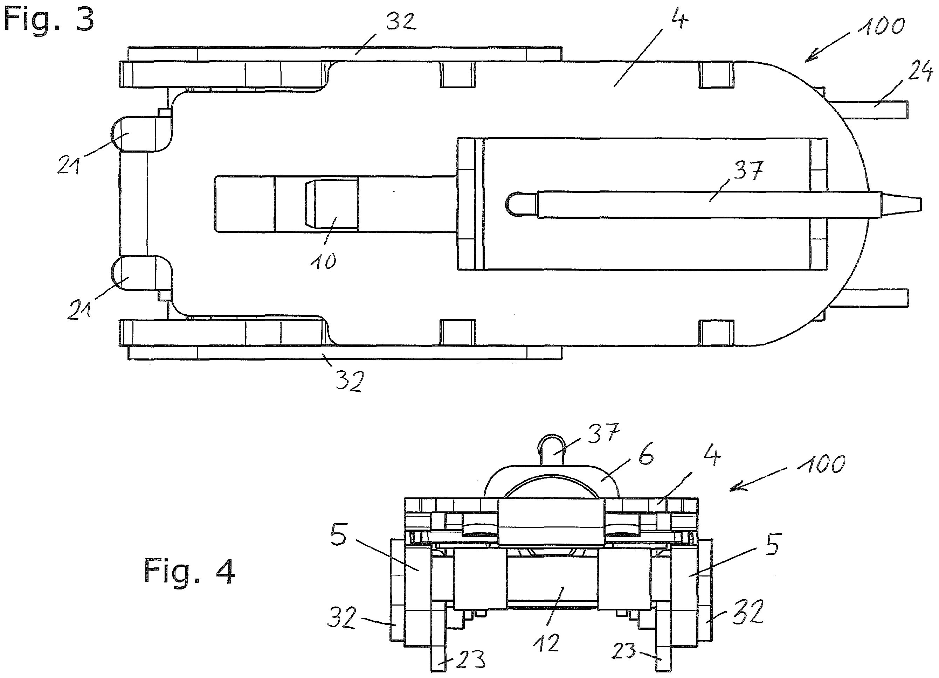

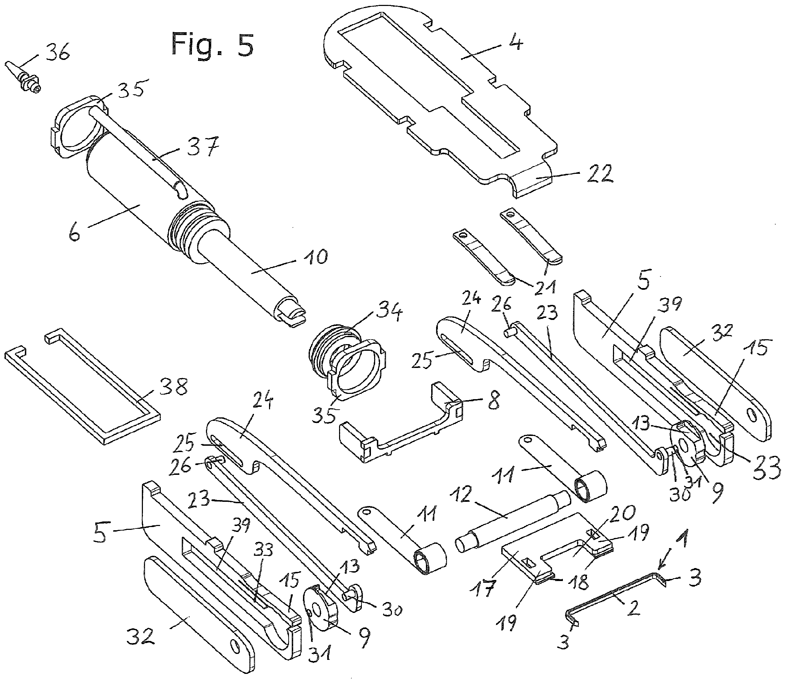

Die Fig. 1 bis Fig. 5 zeigen den Kopfteil 100 einer erfindungsgemäßen Vorrichtung, die eine Gewebeklammer 1 mit einem linearen Hauptabschnitt 2 und zwei davon senkrecht abstehenden, parallelen Eingriffsabschnitten 3 (ersichtlich in Fig. 5) ausstoßen und verformen kann.

Die Vorrichtung weist ein Trägerelement 4 auf, an dem zwei Seitenteile 5, ein Hydraulikzylinder 6 mit einer Kolbenstange 10 und ein Rückhalteelement 22 angeordnet sind. Am Trägerelement 4 liegen Gewebeklammern 1 mit ihren Eingriffsabschnitten 3 in einer Vorratsstellung 7 auf, was in Fig. 2 teilweise dargestellt ist.

Die Seitenteile 5 besitzen jeweils eine Ausnehmung 39, deren Hauptteil rechteckig ist und dazu bestimmt ist, die Eingriffsabschnitte 3 der Gewebeklammern 1 in der Vorratsstellung 7 aufzunehmen und oben und unten zu führen. Außerdem wird in der Ausnehmung 39 das jeweilige Rotorelement 9 aufgenommen.

Die Gewebeklammern 1 liegen in Vorratsstellung 7 im Wesentlichen als Stapel aufeinander, wobei die Ebenen 7a, in denen der der Hauptabschnitt 2 und die Eingriffsabschnitte 3 liegen, parallel zueinander sind. Darüber hinaus liegen die Ebenen 7a im Wesentlichen senkrecht auf die Achse 6a des Hydraulikzylinders 6, die auch gleichzeitig die Längsachse der Vorrichtung darstellt. Dies ermöglicht es, dass die Gewebeklammern 1 in Vorratsstellung 7 so angeordnet sein können, dass sich in dem Raum, der zwischen den Eingriffsabschnitten 3 der Gewebeklammern 1 in Vorratsstellung 7 gebildet wird, zumindest teilweise der Hydraulikzylinder 6 bzw. die Kolbenstange 10 befinden können. Dies ermöglicht einen sehr kompakten und platzsparenden Aufbau.

Die Gewebeklammern 1 in Vorratsstellung 7 werden von einem Vorratsschlitten 8 gegen zwei Rotorelemente 9 gedrückt, welche mit einer Welle 12 verbunden sind. Die dafür notwendige Vorspannung wird durch zwei Vorratsfedern 11 erzeugt. Diese sind als abgewickelte Spiralfedern ausgebildet, je ein Ende ist an der Welle 12 befestigt, die anderen Enden am Vorratsschlitten 8.

Jedes der beiden Rotorelemente 9 ist drehbar gelagert und weist eine Auflagefläche 13 mit einer Auflagenase 16 für einen Eingriffsabschnitt 3 einer Gewebeklammer 1 und eine Vertiefung 14 auf. In die Vertiefung 14 kann eine Rotorfeder 15 eingreifen, um ein Einrasten zu ermöglichen, um Positionen der Rotorelemente 9 zu definieren. Die Rotorfeder 15 ist als Fortsatz des Seitenteils 5 ausgebildet.

Ein Schieber 17 ist an der Kolbenstange 10 befestigt und weist zwei Vorschubbereiche 19 mit Nasen 18 auf. Zwischen den Vorschubbereichen 19 ist eine mittige, rechteckige Ausnehmung 20. Über den Hydraulikzylinder 6 ist der Schieber 17 entlang der Längsachse 6a verschiebbar.

In einer ersten Orientierung der Rotorelemente 9 zeigen die Auflageflächen 13 in Richtung der Gewebeklammern 1 in Vorratsstellung 7. Der Vorratsschlitten 8 drückt dabei eine erste Gewebeklammer 1 an die Auflageflächen 13. Nun können die Rotorelemente 9 um etwa 90° gedreht werden. Dabei wird die aufliegende Gewebeklammer 1 durch die Auflagenasen 16 mitgedreht, um eine Arbeitsstellung 29 zu erreichen, in der sie in einer weiteren Ebene 29a angeordnet ist, die parallel zur Längsachse 6a ist. Beim ersten Teil der Drehung wird die erste Gewebeklammer 1 durch den Vorratsschlitten 8 gegen die Auflageflächen 13 gepresst, beim zweiten Teil der Drehung wird die Flächenpressung durch die Rotorfeder 15 aufrechterhalten, um die erste Gewebeklammer 1 in einer definierten Position zu halten. Der Hydraulikzylinder 6 befindet sich zu diesem Zeitpunkt in einer eingefahrenen Position, der Schieber 17 befindet sich daher hinter dem Hauptabschnitt 2 der ersten Gewebeklammer 1.

Nun kann der Hydraulikzylinder 6 ausgefahren werden. Dabei nimmt der Schieber 17 die erste Gewebeklammer 1 mit. Die Position am Schieber 17 wird gesichert, indem eine Auswurffeder 21 die erste Gewebeklammer 1 gegen die Nase 18 drückt. Gleichzeitig werden durch den Vorschub die Auswurffedern 21 immer stärker vorgespannt.

Die erste Gewebeklammer 1 wird nun gegen das Rückhalteelement 22 gedrückt, welches mittig und an die Breite der Ausnehmung 20 des Schiebers 17 angepasst ist. Dadurch wird die erste Gewebeklammer 1 am Hauptabschnitt 2 zu einem Rechteck verformt.

Bei anschließendem Einfahren des Hydraulikzylinders 6 wird die Vorspannung der Auswurffeder 21 auf die erste Gewebeklammer 1 übertragen und somit vom Rückhalteelement 22 abgestreift.

Die Drehung der Rotorelemente 9 erfolgt je über ein an ihnen angelenktes Pleuel 23, das einen ersten Zapfen 30 aufweist, der in eine exzentrische Bohrung 31 des jeweiligen Rotorelements 9 eingreift. Je ein mit dem Schieber 17 verbundener Verbindungsschieber 24 weist ein Langloch 25 auf, in welches ein zweiter Zapfen 26 des Pleuels 23 eingreift. Durch die Verbindung in einem Langloch 25 wird erreicht, dass die Drehung der Rotorelemente 9 jeweils in einer zweiten Phase der Bewegung des Schiebers 17 erfolgt.

In der Folge werden die weiteren Bauteile der erfindungsgemäßen Vorrichtung kurz erläutert:
Abdeckplatten 32 sind seitlich außerhalb der Seitenteile 5 angebracht, um deren Ausnehmungen 33 abzudecken. Die Kolbenstange 10 ist über Dichtungen 34 gegenüber dem Hydraulikzylinder 6 abgedichtet, der über Montageelemente 35 am Trägerelement 4 befestigt ist. Hydraulikanschlüsse 36 und 37 dienen zur Zufuhr des Hydraulikmediums, um die Vorrichtung zu steuern. Mit 38 ist eine Befestigungsklammer bezeichnet.

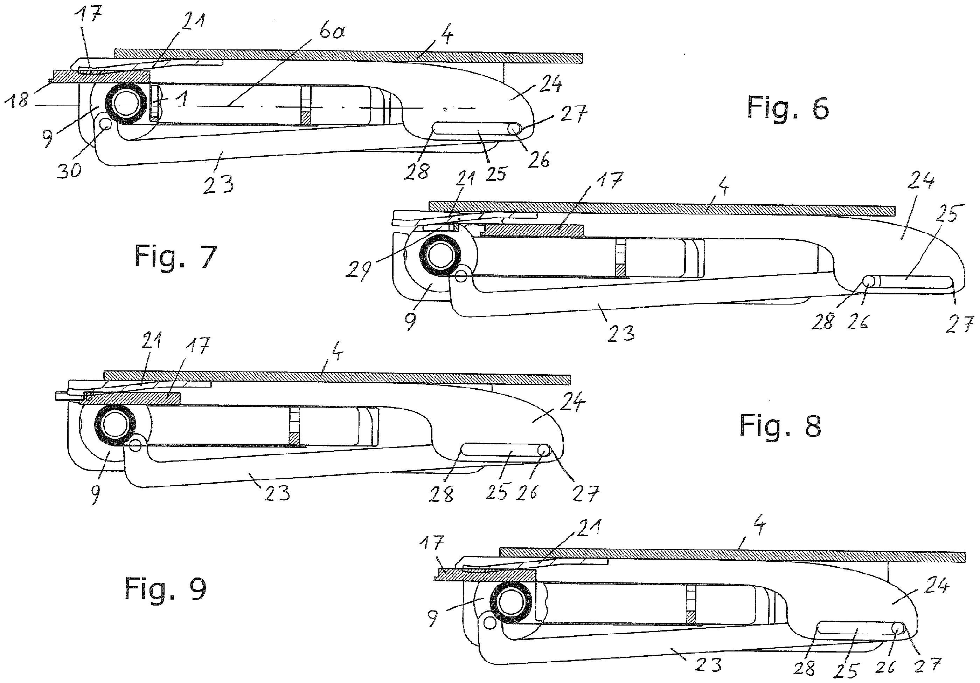

Zur Veranschaulichung des Arbeitsvorgangs der Vorrichtung ist in den Fig. 6 bis 9 jeweils der gleiche Längsschnitt in unterschiedlichen Positionen des Hydraulikzylinders 6 dargestellt.

Fig. 6 zeigt den Ausgangszustand mit Hydraulikzylinder 6 mit vollständig ausgefahrener Kolbenstange 10. Der Zapfen 26 befindet sich im ausfahrseitigen Anschlag 27 des Langlochs 25, die Auflagefläche 13 der Rotorelemente 9 ist parallel zur Längsachse 6a. Der Schieber 17 befindet sich in der vollständig ausgefahrenen Position, in der gerade eine hier nicht dargestellte Gewebeklammer 1 ausgestoßen worden ist.

Ausgehend von dieser Stellung wird die Kolbenstange 10 eingefahren, der Schieber 17 bewegt sich in der Darstellung nach rechts, der mit ihm verbundene Verbindungsschieber 24 bewegt sich ebenfalls mit. Im ersten Teil des Ausfahrvorgangs wird keine Bewegung auf das Pleuel 23 übertragen, da sich der Zapfen 26 in Richtung des einfahrseitigen Anschlags 28 des Langlochs 25 des Verbindungsschiebers 24 bewegt. Nach etwa dem halben Hub ist der einfahrseitige Anschlag 28 erreicht, und das Pleuel 23 wird nun mit dem Schieber 17 und dem Verbindungsschieber 24 mitbewegt. Dadurch werden die Rotorelemente 9 um etwa einen rechten Winkel gedreht, so dass eine erste Gewebeklammer 1, die in Vorratsstellung auf der Auflageflächen 13 aufliegt aus einer in Fig. 6 senkrechten Stellung in eine waagrechte Stellung gedreht wird, in der sie in der Ebene des Schiebers 17 liegt. Durch die Auflagenasen 16 der Rotorelemente 9 wird die an den Auflageflächen 13 aufliegende Gewebeklammer 1 mitgenommen. Da sich der Schieber 17 zu diesem Zeitpunkt bereits hinter die Rotorelemente 9 zurückgezogen hat, ist die Bewegungsbahn für die Gewebeklammer 1 frei.

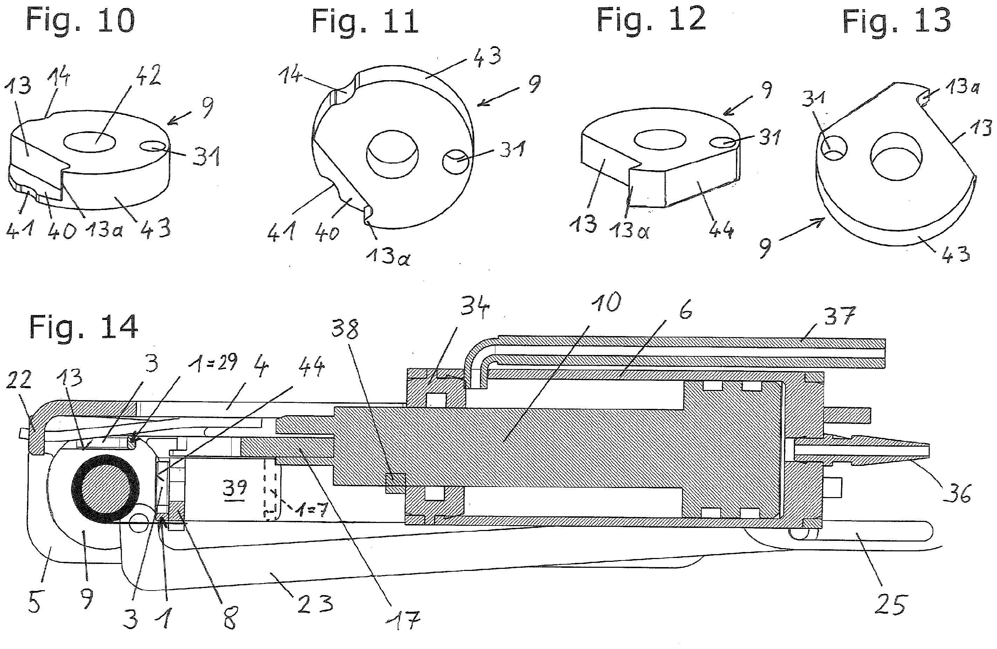

Die so erreichte Stellung mit vollständig eingefahrener Kolbenstange 10 ist in Fig. 7 gezeigt. Nun kann die Kolbenstange 10 wieder aus dem Hydraulikzylinder 6 ausgefahren werden. Es bewegen sich wieder der Schieber 17 mit den Verbindungsschiebern 24 in Richtung der Position der Fig. 6. Dabei wird wieder anfänglich keine Bewegung auf die Pleuel 23 übertragen, da der Zapfen 26 im Langloch 25 in Richtung des ausfahrseitigen Anschlags 27 geschoben wird. Ist der ausfahrseitige Anschlag 27 erreicht, was in der Fig. 8 dargestellt ist, bewegen sich die Pleuel 23 mit der Kolbenstange 10 mit, wodurch die Rotorelemente 9 wieder in die Stellung aus Fig. 6 zurückgedreht werden, die der in der Fig. 10 dargestellten Stellung entspricht. Während der Ausfahrbewegung der Kolbenstange 10, die von Fig. 7 ausgeht und in Fig. 9 endet, wird der Schieber 17 vorgeschoben um letztlich die in der Phase zwischen Fig. 6 und Fig. 7 eingelegte Gewebeklammer 1 zu verformen und auszustoßen. Die Rotorelemente 9 sind in der in der Fig. 9 gezeigten Stellung wieder in der Position, um eine weitere Gewebeklammer 1 aufzunehmen.

Beim Vorschub des Schiebers 17 werden über die Gewebeklammer 1 in Arbeitsstellung 29 die Auswurffedern 21 vorgespannt, wobei die Nasen 18 am Schieber 17 die Gewebeklammer 1 in Arbeitsstellung 29 unten abstützen, so dass die Gewebeklammer 1 beidseitig geführt ist. Beim Zurückziehen des Schiebers 17 entfällt die Abstützung der Gewebeklammer 1 von unten, so dass die Auswurffedern 21 diese nach unten drücken und vom Rückhalteelement 22 abstreifen.

Der Arbeitszyklus kann nach Erreichen der in der Fig. 9 gezeigten Stellung neu beginnen.

In den Fig. 10 bis Fig. 13 sind zwei unterschiedliche Ausführungsvarianten eines Rotorelements 9 dargestellt. Die erste Ausführungsvariante, die in den Fig. 10 und Fig. 11 dargestellt ist, entspricht dabei dem in den Ausführungsvarianten der Fig. 1 bis Fig. 9 verwendeten und dargestellten Rotorelement 9.

Diese erste Ausführungsvariante besitzt eine Auflagefläche 13, die einen Teil eines Kreissegments in Bezug auf den Umfang 43 bildet. An einem Ende ist eine Nase 13a vorgesehen, um die Gewebeklammer 1, die in ihrer Vorratsstellung mit ihrem Eingriffsabschnitt 3 an der Auflagefläche 13 anliegt, bei der Drehung des Rotorelements 9 mitzunehmen. Die Auflagefläche 13 erstreckt sich nicht über die gesamte Dicke des Rotorelements 9. an einer Seite bleibt ein Steg 40 stehen, der den Eingriffsabschnitt 3 der anliegenden Gewebeklammer 1 seitlich führt. Im Bereich dieses Stegs 40 ist eine erste Vertiefung 41 vorgesehen und vom Mittelpunkt des Rotorelementes 9 aus gesehen rechtwinklig dazu ist eine zweite Vertiefung 14 im Umfang 43 ausgebildet. Die beiden Vertiefungen 41, 14 dienen dazu, dass die Rotorfeder 15 einrastet und die Endpositionen der Verdrehung des Rotorelements 9 festlegt. Die exzentrische Bohrung 31 dient der Verdrehung durch das Pleuel 23. Die Bohrung 42 ist zum Aufstecken auf die Welle 12 vorgesehen.

Bei der alternativen Ausführungsvariante, die in den Fig. 12 und Fig. 13 dargestellt ist, erstreckt sich die Auflagefläche 13 über die gesamte Dicke des Umfangs 43. Die seitliche Führung wird dabei von den Abdeckplatten 32 übernommen. Zusätzlich zu der Auflagefläche 13 ist bevorzugt rechtwinkelig dazu eine weitere Anlagefläche 44 am Umfang vorgesehen, an der die Gewebeklammern 1 in der Vorratsstellung 7 anliegen, wenn die Auflagefläche 13 in die Auswurfstellung (parallel zu der Längsachse 6a) gedreht ist. Bei dieser Ausführungsvariante definieren die an der Auflagefläche 13 bzw. der Anlagefläche 44 anliegenden und durch die Vorratsfedern 11 angedrückten Eingriffsabschnitte 3 der Gewebeklammern 1 die Endpositionen der Verdrehung des Rotorelements 9. Vertiefungen sind daher hier nicht erforderlich.

Fig. 14 zeigt einen Längsschnitt einer Ausführungsvariante der erfindungsgemäßen Vorrichtung unter Verwendung der Rotorelemente 9 der Fig. 12 und Fig. 13. Diese Variante entspricht weitgehend der in den Fig. 1 bis Fig. 9 dargestellten Ausführungsvariante. Es ist ersichtlich, dass der Eingriffsabschnitt 3 einer ersten Gewebeklammer 1 auf der Auflagefläche 13 bereits in in der Arbeitsstellung 29 vorliegt. Eine weitere Gewebeklammer 1 liegt mit ihrem Eingriffsabschnitt 3 an der weiteren Anlagefläche 44 an und wird vom Vorratsschieber 8 angedrückt, so dass das Rotorelement 9 in dieser Stellung federnd fixiert ist. Nach dem Auswerfen der ersten Gewebeklammer 1 wird das Rotorelement 9 im Uhrzeigersinn zurückgedreht, wobei jedoch der Vorratsschieber 8 samt den im Vorratsstellung 7 befindlichen Gewebeklammern 1 zunächst gegen den Widerstand der Vorratsfedern 11 zurückgedrückt werden muss.

Aus Fig. 14 ist auch ersichtlich, dass sich die Eingriffsabschnitte 3 der Gewebeklammern 1 im Vorratsstellung 7 (eine Gewebeklammer 1 ist mit unterbrochenen Linien entsprechend dargestellt) deutlich über den unteren Bereich der Kolbenstange 10 hinaus nach oben erstrecken, was bedeutet, dass sich die Kolbenstange 10 in der ausgefahrenen Stellung innerhalb des rechteckigen Raums bewegt, der an drei Seiten von dem Hauptabschnitt 2 und den beiden Eingriffsabschnitten 3 der Gewebeklammer 1 aufgespannt ist.

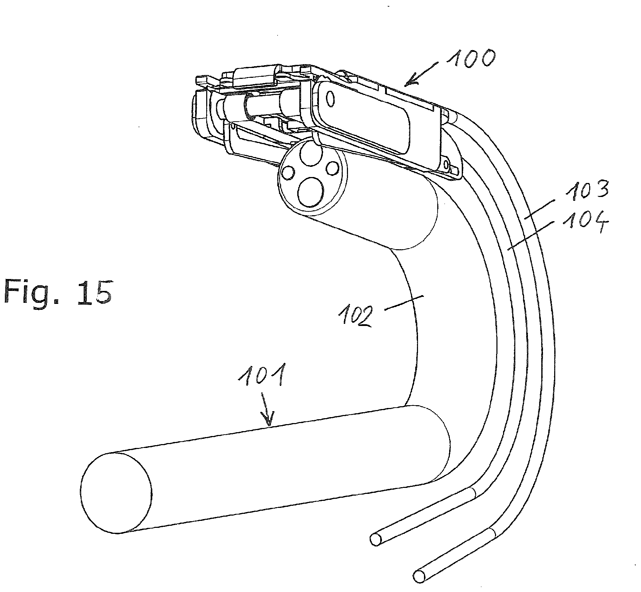

In Fig. 15 ist eine erste Variante des Einsatzes einer erfindungsgemäßen Vorrichtung dargestellt. Dabei ist der Kopfteil 100 abnehmbar auf einem üblichen Endoskop 101 mit einer Abwinkelung 102 aufgesetzt und wird über Hydraulikschläuche 103 und 104 gesteuert, die parallel zum Endoskop in eine Körperöffnung eingeführt werden.

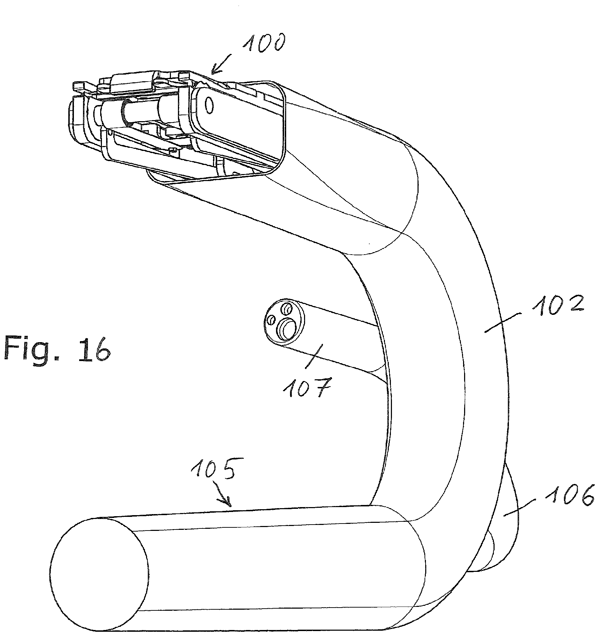

Fig. 16 zeigt eine alternative Variante des Einsatzes der erfindungsgemäßen Vorrichtung, bei der der Kopfteil 100 die Spitze eines eigenständigen flexiblen Instruments 105 bildet. Dieses besitzt gemäß üblicher Bauart mindestens einen Kanal, durch den ein Endoskop 107 mit einer Abwinkelung 102 einschiebbar ist. Das Endoskop 107 ist mit einer eigenständigen Abwinkelung 106 seitlich herausgeführt, um den Vorgang des Verklammerns beobachten zu können.

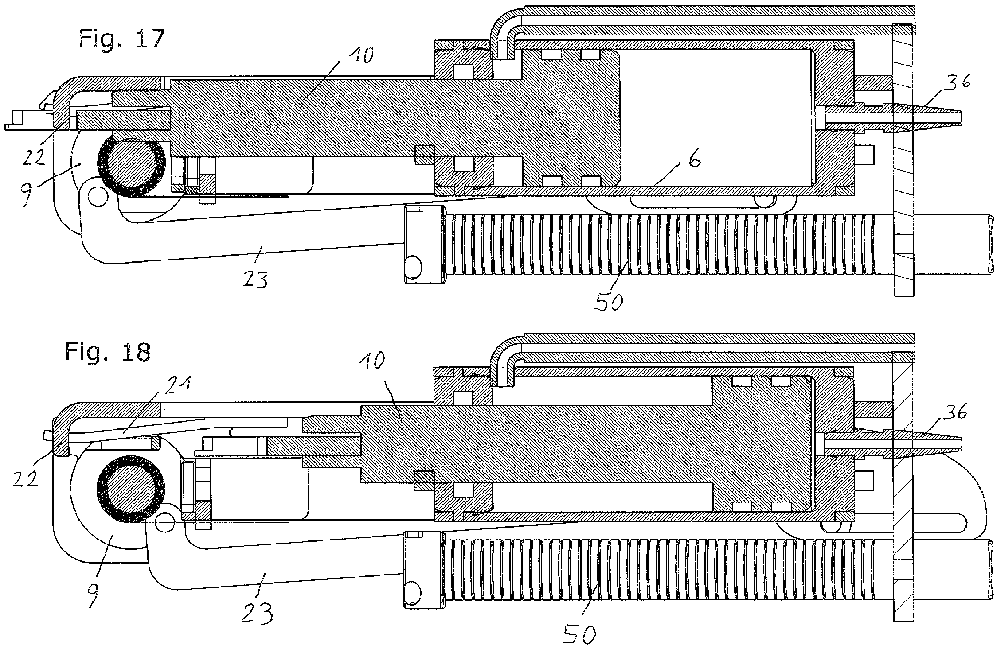

Die Ausführungsvariante der Fig. 17 und Fig. 18 unterscheidet sich von den oben beschriebenen Varianten dadurch, dass zwei Kanäle 50, 51 unterhalb des Hydraulikzylinders 6 vorgesehen sind. Die Kanäle 50, 51 sind zum distalen Ende hin nach außen gerichtet, also divergent, wobei der Winkel der Divergenz vorzugsweise einstellbar ist. Wenn nun durch die Kanäle 50 und 51 jeweils Halteinstrumente vorgeschoben werden, dann treten diese leicht nach außen gerichtet aus, wobei sich somit variable Ausmaß durch den Abstand zur Spitze, also den Raum unterhalb der Gewebeklammern 1 in ihrer Vorratsstellung 7 noch vergrößert.

Durch die besondere Gestaltung der Vorrichtung ist es möglich die Kanäle 50 und 51 innerhalb des kreisförmigen Querschnitts anzuordnen, der durch die übrigen Bauteile definiert ist.

Die vorliegende Erfindung ermöglicht es, Gewebe im Körperinneren mit Gewebeklammern zu verbinden, wobei es besonders vorteilhaft ist, dass nacheinander eine Mehrzahl von Gewebeklammern angewendet werden können, ohne die Notwendigkeit, die Vorrichtung dazu aus dem Körper des Patienten zu ziehen und danach erneut einzuführen.

## Patentansprüche

1. Vorrichtung zum Verbinden von Körpergeweben mit einem in eine Körperöffnung einschiebbaren Kopfteil (100) mit einer Längsachse (6a), wobei in dem Kopfteil (100) eine Mehrzahl von Gewebeklammern (1) in einer Vorratsstellung (7) aufgenommen sind, die aus einem linearen Hauptabschnitt (2) und zwei davon senkrecht abstehenden Eingriffsabschnitten (3) bestehen und die jeweils in einer ersten Ebene (7a) ausgerichtet sind, die im Wesentlichen senkrecht zur Längsachse (6a) ist, und mit einen Hydraulikzylinder (6) und einer Kolbenstange (10), die mit einem Schieber (17) fest verbunden ist, **dadurch gekennzeichnet, dass** der Hydraulikzylinder (6) und/oder die Kolbenstange (10) zumindest teilweise im Inneren des Raums angeordnet sind, der zwischen den Eingriffsabschnitten (3) der Gewebeklammern (1) in der Vorratsstellung (7) liegt und dass zwei Rotorelemente (9) vorgesehen sind, die um eine zu den Hauptabschnitten (2) der Gewebeklammern (2) parallele Achse schwenkbar sind und die jeweils eine Auflagefläche (13) für einen Eingriffsabschnitt (3) einer Gewebeklammer (1) aufweisen, um diese aus der Vorratsstellung (7) in eine Arbeitsstellung (29) zu drehen, in der die Gewebeklammer (1) in einer weiteren Ebene (29a) angeordnet ist, die im Wesentlichen senkrecht zur ersten Ebene (7a) orientiert ist, und dass ein Schieber (7) vorgesehen ist, um die in der Arbeitsstellung (29) vorliegende Gewebeklammer (1) in der weiteren Ebene (29a) vorzuschieben und in eine Klammerstellung zu verformen.

2. Vorrichtung nach Anspruch 1, **dadurch gekennzeichnet, dass** der Hydraulikzylinder (6) doppelwirkend ausgeführt ist.

3. Vorrichtung nach einem der Ansprüche 1 bis 2, **dadurch gekennzeichnet, dass** jeweils eine Rotorfeder (15) an einem Rotorelement (9) angreift, um dessen Bewegung in zwei Rotationsstellungen einrastbar auszuführen, und dass vorzugsweise die Rotorelemente (9) mit einer Welle (12) miteinander verbunden sind.

4. Vorrichtung nach einem der Ansprüche 1 bis 3, **dadurch gekennzeichnet, dass** die Rotorelemente (9) durch zumindest ein Pleuel (23) angetrieben werden, das an ihnen angelenkt ist, und welches vorzugsweise über einen Verbindungsschieber (24) mit dem Schieber (17) in Verbindung steht.

5. Vorrichtung nach Anspruch 4, **dadurch gekennzeichnet, dass** der bzw. die Verbindungsschieber (24) fest mit dem Schieber (17) verbunden ist/sind, und dass an jedem Verbindungsschieber (24) ein Langloch (25) angeordnet ist, in das je ein Zapfen (26) des Pleuels (23) eingreift.

6. Vorrichtung nach einem der Ansprüche 1 bis 5, **dadurch gekennzeichnet, dass** die Gewebeklammern (1) in Vorratsstellung (7) in Ausnehmungen (39) von Seitenteilen (5) geführt sind.

7. Vorrichtung nach einem der Ansprüche 1 bis 6, **dadurch gekennzeichnet, dass** der Schieber (17) auf der, den Eingriffsabschnitten (3) abgewandten, Seite der Hauptabschnitte (2) der Gewebeklammern (1) in Richtung der Längsachse (6a) beweglich angeordnet ist, der an seiner vorderen Stirnseite zwei seitliche Vorschubbereiche (19) aufweist, zwischen denen eine Ausnehmung (20) vorgesehen ist, und dass ein Rückhalteelement (22) vorgesehen ist, das in die Ausnehmung (20) des Schiebers (17) eingreift, um beim Vorschub des Schiebers (17) den linearen Hauptabschnitt (2) einer dazwischenliegenden Gewebeklammer (1) zu verformen.

8. Vorrichtung nach einem der Ansprüche 1 bis 7, **dadurch gekennzeichnet, dass** der Schieber (17) plattenförmig ausgebildet ist und der Bewegungsbereich des Schiebers (17) in einer weiteren Ebene (29a) liegt, die im Bereich der Spitzen der Eingriffsabschnitte (3) der Gewebeklammern (1) in Vorratsstellung (7) liegt, und besonders vorzugsweise im Wesentlichen parallel und mit konstantem Abstand zu den Hauptabschnitten (2) der Gewebeklammern (1) in Vorratsstellung (7) liegt.

9. Vorrichtung nach einem der Ansprüche 1 bis 8, **dadurch gekennzeichnet, dass** der Schieber (17) eine vordere Stellung und eine hintere Stellung besitzt, wobei in der vorderen Stellung zwischen dem Schieber (17) und dem Rückhalteelement (22) eine verformte Gewebeklammer (1) eingeklemmt ist, und in der hinteren Stellung eine, aus der Vorratsstellung (7) in eine senkrecht dazu liegende Arbeitsstellung (29) gedrehte Gewebeklammer (1) aufnehmbar ist.

10. Vorrichtung nach einem der Ansprüche 1 bis 9, **dadurch gekennzeichnet, dass** der Kopfteil (100) als Aufsatz auf ein Endoskop (101) ausgebildet ist oder einstückig mit einem flexiblen Instrument (105) verbunden ist, das vorzugsweise einen Kanal für ein Endoskop (107) aufweist.

11. Vorrichtung nach einem der Ansprüche 1 bis 10, **dadurch gekennzeichnet, dass** mindestens ein Kanal (50, 51) zur Aufnahme eines Halteinstruments vorgesehen ist.

12. Vorrichtung nach Anspruch 12, **dadurch gekennzeichnet, dass** zwei Kanäle (50, 51) vorgesehen sind, die vorzugsweise nach vorne geringfügig auseinanderlaufen, wobei besonders vorzugsweise der Winkel zwischen den Kanälen (50, 51) steuerbar ist.

13. Vorrichtung nach einem der Ansprüche 11 oder 12, **dadurch gekennzeichnet, dass** die Halteinstrumente unabhängig voneinander steuerbar sind.

14. Vorrichtung nach einem der Ansprüche 11 bis 13, **dadurch gekennzeichnet, dass** der Kanal (50, 51) benachbart zum Hydraulikzylinder (6), und/oder zwischen den Seitenteilen (5), und/oder im proximalen Bereich des Kopfteils (100) angeordnet ist.

## Claims

1. Device for connecting body tissues, having a head part (100), which can be pushed into a body opening and has a longitudinal axis (6a), wherein a plurality of tissue staples (1) are accommodated in the head part (100) in a storage position (7), which tissue staples consist of a linear main section (2) and two engagement sections (3) projecting perpendicularly therefrom and are each aligned in a first plane (7a) which is substantially perpendicular to the longitudinal axis (6a), and having a hydraulic cylinder (6) and a cylinder rod (10) which is fixedly connected to a slider (17), **characterised in that** the hydraulic cylinder (6) and/or the cylinder rod (10) are arranged at least partially inside the space which lies between the engagement sections (3) of the tissue staples (1) in the storage position (7), and **in that** two rotor elements (9) are provided, which are pivotable about an axis parallel to the main sections (2) of the tissue staples (2) and which each have a bearing surface (13) for an engagement section (3) of a tissue staple (1) in order to rotate the said tissue staple from the storage position (7) into a working position (29), in which the tissue staple (1) is arranged in a further plane (29a), which is oriented substantially perpendicularly to the first plane (7a), and **in that** a slider (7) is provided in order to advance the tissue staple (1) which is present in the working position (29) in the further plane (29a) and to deform it into a clamping position.

2. Device according to claim 1, **characterised in that** the hydraulic cylinder is of double-acting design.

3. Device according to one of claims 1 to 2, **characterised in that** a rotor spring (15) engages in each case on a rotor element (9) in order to carry out its movement in two rotational positions so as to be latchable, and **in that** the rotor elements (9) are preferably connected to one another by means of a shaft (12).

4. Device according to one of claims 1 to 3, **characterised in that** the rotor elements (9) are driven by at least one connecting rod (23) which is articulated to them and which is preferably connected to the slider (17) via a connecting slider (24).

5. Device according to claim 4, **characterised in that** the connecting slider or sliders (24) is or are firmly connected to the slider (17), and **in that** an elongated hole (25) is arranged on each connecting slider (24), in each of which a pin (26) of the connecting rod (23) engages.

6. Device according to one of claims 1 to 5, **characterised in that** the tissue staples (1) are guided, in the storage position (7), in recesses (39) of side parts (5).

7. Device according to one of claims 1 to 6, **characterised in that** the slider (17) is arranged on the side of the main sections (2) of the tissue staples (1) facing away from the engagement sections (3) so as to be movable in the direction of the longitudinal axis (6a), said slider having two lateral feed regions (19) on its front end face, between which a recess (20) is provided, and **in that** a retaining element (22) is provided which engages in the recess (20) of the slider (17) in order to deform the linear main section (2) of an interposed tissue staple (1) when the slider (17) is advanced.

8. Device according to one of the claims 1 to 7, **characterised in that** the slider (17) is plate-shaped and the range of movement of the slider (17) lies in a further plane (29a) which lies in the storage position (7) in the region of the tips of the engagement sections (3) of the tissue staples (1) and lies particularly preferably substantially parallel and at a constant distance from the main sections (2) of the tissue staples (1) in the storage position (7).

9. Device according to one of the claims 1 to 8, **characterised in that** the slider (17) has a front position and a rear position, wherein a deformed tissue staple (1) is clamped between the slider (17) and the retaining element (22) in the front position, and a tissue staple (1) turned from the storage position (7) into a working position (29) disposed perpendicular thereto is receivable in the rear position.

10. Device according to one of claims 1 to 9, **characterised in that** the head part (100) is designed as an attachment to an endoscope (101) or is integrally connected to a flexible instrument (105) which preferably has a channel for an endoscope (107).

11. Device according to one of claims 1 to 10, **characterised in that** at least one channel (50, 51) is provided for receiving a holding instrument.

12. Device according to claim 12, **characterised in that** two channels (50, 51) are provided which preferably diverge slightly towards the front, wherein the angle between the channels (50, 51) is particularly preferably controllable.

13. Device according to one of claims 11 or 12, **characterised in that** the holding instruments are controllable independently of one another.

14. Device according to one of claims 11 to 13, **characterised in that** the channel (50, 51) is arranged adjacent to the hydraulic cylinder (6), and/or between the side parts (5), and/or in the proximal region of the head part (100).

## Revendications

1. Dispositif pour relier des tissus corporels à une partie (100) de tête, pouvant être insérés dans un orifice du corps, ayant un axe (6a) longitudinal, dans lequel dans la partie (100) de tête sont reçues une pluralité d'agrafes (1) pour du tissu en une position (7) de réserve, qui est constituée d'une partie (2) linéaire principale et de deux parties (3) de pénétration s'en éloignant perpendiculairement et qui sont dirigées respectivement dans un premier plan (7a), qui est sensiblement perpendiculaire à l'axe (6a) longitudinal, et comprenant un vérin (6) hydraulique et une tige (10) de piston, qui est assemblée fixement à un tiroir (17), **caractérisé en ce que** le vérin (6) hydraulique et/ou la tige (10) de piston sont disposés au moins en partie à l'intérieur de l'espace, qui se trouve entre les parties (3) de pénétration des agrafes (1) pour du tissus dans la position (7) de réserve et **en ce qu'**il est prévu deux éléments (9) de rotor, qui peuvent pivoter autour d'un axe parallèle aux parties (2) principales des agrafes (2) pour du tissu et qui ont chacun une surface (13) de support pour une partie (3) de pénétration d'une agrafe (1) pour du tissu, afin de faire tourner celle-ci de la position (7) de réserve à une position (29) de travail, dans laquelle l'agrafe (1) pour du tissu est disposée dans un autre plan (29a), qui est orienté sensiblement perpendiculairement au premier plan (7a), et **en ce qu'**il est prévu un tiroir (7), afin d'avancer dans l'autre plan (29a) l'agrafe (1) pour du tissu présente dans la position (29) de travail et la déformer en une position d'agrafage.

2. Dispositif suivant la revendication 1, **caractérisé en ce que** le vérin (6) hydraulique est à double effet.

3. Dispositif suivant l'une des revendications 1 à 2, **caractérisé en ce que** respectivement un ressort (15) de rotor attaque un élément (9) de rotor, afin d'exécuter, avec possibilité d'encliquetage, son déplacement dans deux positions en rotation et **en ce que** de préférence les éléments (9) de rotor sont assemblés entre eux par un arbre (12).

4. Dispositif suivant l'une des revendications 1 à 3, **caractérisé en ce que** les éléments (9) de rotor sont entraînés par au moins une bielle (23), qui y est articulée et qui est en liaison avec le tiroir (17) de préférence par un tiroir (24) de liaison.

5. Dispositif suivant la revendication 4, **caractérisé en ce que** le ou les tiroirs (24) de liaison est/sont assemblés d'une manière fixe au tiroir (17), et **en ce que** sur chaque tiroir (24) de liaison est disposé une boutonnière (25) dans laquelle pénètre un tenon (26) de la bielle (23).

6. Dispositif suivant l'une des revendications 1 à 5, **caractérisé en ce que** les agrafes (1) pour du tissu sont guidées dans la position (7) de réserve dans des évidements (39) de parties (5) latérales.

7. Dispositif suivant l'une des revendications 1 à 6, **caractérisé en ce que** le tiroir (17) est, du côté, non tourné vers les parties (3) de pénétration, des parties (2) principales des agrafes (1) pour du tissu, monté mobile dans la direction de l'axe (6a) longitudinal, lequel a du côté frontal avant deux parties (19) latérales d'avance, entre lesquelles est prévu un évidement (20), et **en ce qu'**il est prévu un élément (22) de retenu, qui pénètre dans l'évidement (20) du tiroir (17), afin de déformer, lors de l'avance du tiroir (17), la partie (2) principale linéaire d'une agrafe (1) pour du tissu interposée.

8. Dispositif suivant l'une des revendications 1 à 7, **caractérisé en ce que** le tiroir (17) est constitué sous la forme d'une plaque et la partie de déplacement du tiroir (17) se trouve dans un autre plan (29a), qui se trouve dans la partie des pointes des parties (3) de pénétration des agrafes (1) pour du tissu dans la position (7) de réserve et, en particulier, de préférence sensiblement parallèlement à une distance constante des parties (2) principales des agrafes (1) pour du tissu dans la position (7) de réserve.

9. Dispositif suivant l'une des revendications 1 à 8, **caractérisé en ce que** le tiroir (17) possède une position avant et une position arrière, dans lequel, dans la position avant, une agrafe (1) pour du tissu déformée est serrée entre le tiroir (17) et l'élément (22) de retenu et, dans la position arrière, une agrafe (1) pour du tissu, tournée de la position (7) de réserve à une position (29) de travail perpendiculaire à celle-ci, peut être reçue.

10. Dispositif suivant l'une des revendications 1 à 9, **caractérisé en ce que** la partie (100) de tête est constituée en coiffe sur un endoscope (101) ou est assemblée d'une pièce avec un instrument (105) souple, qui a de préférence un conduit pour un endoscope (107).

11. Dispositif suivant l'une des revendications 1 à 10, **caractérisé en ce qu'**il est prévu au moins un conduit (50, 51) de réception d'un instrument de maintien.

12. Dispositif suivant la revendication 11, **caractérisé en ce qu'il** est prévu deux conduits (50, 51), qui s'écartent légèrement l'un de l'autre vers l'avant, dans lequel en particulier de préférence l'angle entre les conduits (50, 51) peut être commandé.

13. Dispositif suivant l'une des revendications 11 ou 12, **caractérisé en ce que** les instruments de maintien peuvent être commandés indépendamment l'un de l'autre.

14. Dispositif suivant l'une des revendication 11 à 13, **caractérisé en ce que** le conduit (50, 51) est disposé au voisinage du vérin (6) hydraulique, et/ou entre les parties (5) latérales et/ou dans la partie proximale de la partie (100) de tête.
